(19) Europäisches Patentamt / European Patent Office / Office européen des brevets

(11) EP 2 221 022 A1

(12) EUROPEAN PATENT APPLICATION

(43) Date of publication:
25.08.2010 Bulletin 2010/34

(51) Int Cl.:
A61C 8/00 (2006.01)     B25B 23/142 (2006.01)

(21) Application number: 09167406.9

(22) Date of filing: 06.08.2009

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA RS

(30) Priority: 19.02.2009 IT MI20090225

(71) Applicants:
• Studio A.I.P. S.R.L.
21040 Oggiona con Santo Stefano (VA) (IT)

• 4T Quattroti S.R.L.
21040 Cislago (VA) (IT)

(72) Inventors:
• Brivio, Antonio
21040 Oggiona con Santo Stefano (VA) (IT)
• Brivio, Marco
21040 Oggiona con Santo Stefano (VA) (IT)

(74) Representative: Ponzellini, Gianmarco
Ponzellini, Gioia e Associati S.r.l.
Via Mascheroni, 31
20145 Milano (IT)

(54) **Screwing device for medical applications, particularly for endosseous implantology**

(57)     A screwing device has been realised for applications in endosseous implantology, consisting of a front portion (2) having a torque wrench (3) suitable for acting upon a suitable screw (8) of a dental implant and a rear portion (4) suitable for being manually actuated by the user; the front portion (2) can be separated from the rear portion (4) for sterilisation when the device is not in use.

A sensor (5) is linked to the rear portion (4) in order to detect a deformation thereof resulting from the torque applied by means of the wrench (3). The device also comprises an external module (6) electrically connected to the sensor (5) so as to receive a signal originating from the latter and enable the processing thereof in order to measure the torque applied by means of the wrench (3).

The screwing device is therefore usable in a medical setting, since it is easily sterilisable and does not substantially modify either the wrench or the handgrip, thus allowing the already acquired operating methods to be maintained.

FIG 1

## Description

The present invention relates to a screwing device for medical applications and in particular a screwing device for endosseous implantology.

As is well known, the procedures for reconstructing a tooth or a portion of a dental arch using endosseous implantology techniques involve a surgical step performed by the dentist, which comprises the insertion of an endosseous screw inside the mandibular or maxillary bone of a patient.

This screw is fixed to the patient using a suitable wrench manually actuated by the physician.

It is clear that applying the correct tightening force is extremely important to ensure the success of the implant itself and prevent any type of complication both during the implant insertion phase and during work on the reconstructed structure.

To date, the only system used in medical practice to set a torque threshold value to be reached during the operation of screwing of a dental implant is of a mechanical type and relies on the use of dynamometric wrenches which clicks on reaching the set value.

The above-mentioned dynamometric wrenches are substantially ratchet wrenches with clicks, which can be freely rotated in one direction only and which click in the other direction on exceeding a preset threshold value.

The tool briefly described above, albeit allowing better control of the optimal torque to be applied, presents several major operating limits.

Firstly, due to wear and shifting of internal mechanical components, the precision with respect to the threshold value appears variable over time. Furthermore, the final click effected by the tool on reaching the set value gives rise to rebound effects tied to the internal elasticity.

Finally, it is not possible to keep a documental record of how the tightening operation was performed; if the operation is carried out too abruptly or incorrectly, it may cause phenomena of bone necrosis.

Generally speaking, all types of mechanical dynamometric wrenches based on the same principle are affected by the above-mentioned drawbacks.

Though no valid alternatives to what was briefly described above exist today in the field of endosseous implantology, some of the problems highlighted have also manifested themselves in other types of applications, which regard, however, sectors completely different from the medical sector, for example in precision manual tightening of mechanical components. With the aim of at least partly solving the problems mentioned, wrenches with electronic torque control have been conceived for the specific sectors concerned.

For example, patents US4397196, US7313990 and US2006/27058 describe respective electronic screwing devices which include a torsion sensor (for example defined by one or more strain gauges) suitably positioned in relation to the screwing wrench and a corresponding display for displaying the torque applied or, alternatively, audible or vibratory indicating means for warning that a desired torque value has been reached or preset thresholds have been exceeded.

All of the above-mentioned patents, besides not having found any possible application in the medical field, have revealed to be affected by a major drawback.

In fact, when the point of application of force by the user on the tool handgrip varies in relation to an optimal point of application, a reading error by the tool is generated.

In other words, the actual torque applied being equal, the torque value measured by the sensor varies as a function of the point of application of the force exerted by the user.

It is evident that while such an error may be acceptable for certain applications (mechanical tightening), in others, such as the insertion of screws in a patient's bone, it may have major undesired repercussions.

In the above-described context, an attempt has been made to solve this problem as well according to patent US4864841.

The adoption of an electronic wrench having at least two strain gauges along the direction of extension thereof, as well as the adoption of a correction factor, has rendered the torque reading independent from the point of application of force by the user. Notwithstanding the developments and improvements that have occurred over time, the currently known devices still present several major operating limits and are absolutely unsuitable for use in a medical-dental or surgical setting.

In the medical sector there is a felt need to have a wrench, in particular for endosseous implants, which enables screwing by the application of correct forces and may thus prove to be a tool that is precise and stable over time.

There is likewise a strong need to maintain, to the extent possible, the ease of handling and dimensional characteristics of the mechanical or torque wrench in order to maintain the working approach acquired by medical personnel who operate on patients.

It is in fact extremely important that the physician operating method can remain unchanged and that surgeons can continue to use a tool on which they have built up their experience and for which they have acquired a sensitivity of use such as to optimise the success of the operation. On the contrary, the electronic torque wrenches described above appear to be wholly unsuitable for use in the medical sector, being bulky and awkward to handle and certainly very different from the mechanical wrenches currently used to carry out the aforementioned surgical operations, as well as being difficult to sterilise in accordance with current regulations.

It should be noted, moreover, that the use of instruments in a surgical setting is subject by law to the requirement that all parts of the system that may come into contact with patients be autoclaveable.

The object of the present invention is thus to substantially solve all of the drawbacks tied to the operating limits highlighted above.

It is a first object of the invention to be able to use, in a medical setting, a precise tool whose performance does not change over time.

Furthermore, given the existence in the market of many types of ratchet wrenches, each with its own seat (square, hexagonal, etc.) depending on the dental implant sold, it is a fundamental object of the present invention to be able to reuse the inserts of the old mechanical wrench so that the user can continue to use his or her standard operating procedure.

In other words, in addition to providing a tool that is stable and precise over time, it is an object of the invention to maintain, to the extent possible, the ease of handling and dimensional characteristics of the mechanical torque ratchet wrench, so as not to interfere with the acquired medical practice.

It is a subsidiary object of the invention to make available an intelligent tool, i.e. one that is capable not only of performing the operations for which it was designed, but also of providing substantial evidence of the operations carried out.

Another object of the present invention is to provide a screwing system that is suitable not only in the field of dentistry, but also in the surgical field, by guaranteeing the autoclaveability of all parts of the system that may possibly come into contact with patients.

Finally, it is a further object of the invention to place on the market a tool that can enable fast, simple adaptability to any existing type of insert, thereby assuring an interchangeability of couplings (square, hexagonal, etc...)

These and other objects are substantially achieved by a screwing device for medical applications, in particular for endosseous implants, according to the appended claims.

Further characteristics and advantages of the present invention will become more apparent from the detailed description of several preferred, but not exclusive, embodiments of a screwing device, particularly for dental applications.

Said description is given hereunder with reference to the appended exemplary, and hence non-limiting, drawings, in which:

- Figs. 1 and 1a show a screwing device for medical applications in a first and second embodiment according to the present invention;
- Figs. 2 and 2a respectively show the devices of Figures 1 and 1a in a second operating condition;
- Figs. 3, 4 and 5 show details of the screwing device with an indication of several forces of application and the torque;
- Figs. 3a and 4a illustrate a detail of the second embodiment in a first assembled condition and in a second condition with the wrench elements in a partial exploded view;
- Figs. 6 and 7 show a partial sectional view of the screwing device, respectively from below and from the side;
- Figs. 6a and 7a show a partial sectional view of the alternative embodiment of the screwing device (as per Figures 1a, 2a, 3a and 4a), respectively from below and from the side;
- Fig. 8 shows an exploded view of the device of Figure 7;
- Fig. 9 shows a block wiring diagram of the invention;
- Fig. 9a shows a variant of the block wiring diagram of the invention as per Figure 9, applicable preferably, but not necessarily, to the second embodiment;
- Fig. 10 is a graphical representation that shows the trend in total trim (Y axis) as a function of the point of application of the strain gauges along the device axis (X axis);
- Fig. 11 shows a table indicating the calculation of total trim with the formula stated in Figure 10 and the corresponding real value thereof measured at different points of application of the sensor;
- Fig. 12 illustrates a possible exemplary screen display of the data certifiable using the screwing device according to the invention;
- Fig. 13 shows a table for calculating and checking the values of torque applied and read with the device according to the invention.

With reference to the above-mentioned Figures, 1 indicates an overall screwing device for medical applications, and in particular for applications in endosseous implantology.

In particular, the screwing device is suitable for enabling the engagement, to the maxillary or mandibular bone of patient, of a suitable screw 8 for an implant designed to permit the reconstruction of a tooth or a portion of a dental arch of a patient.

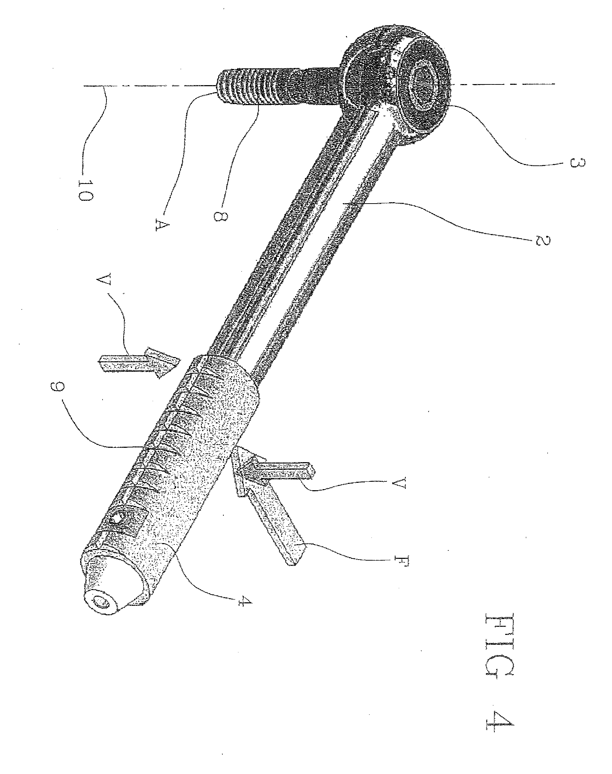

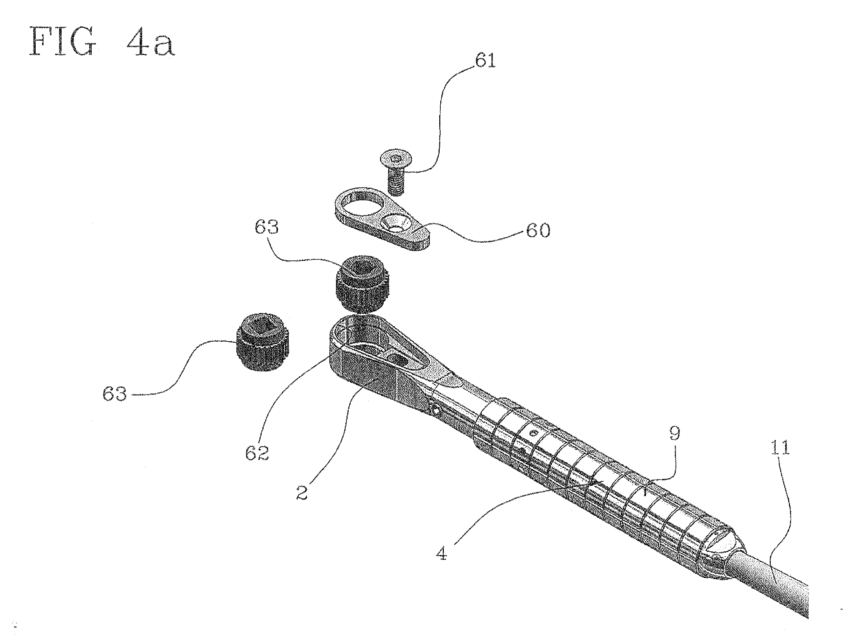

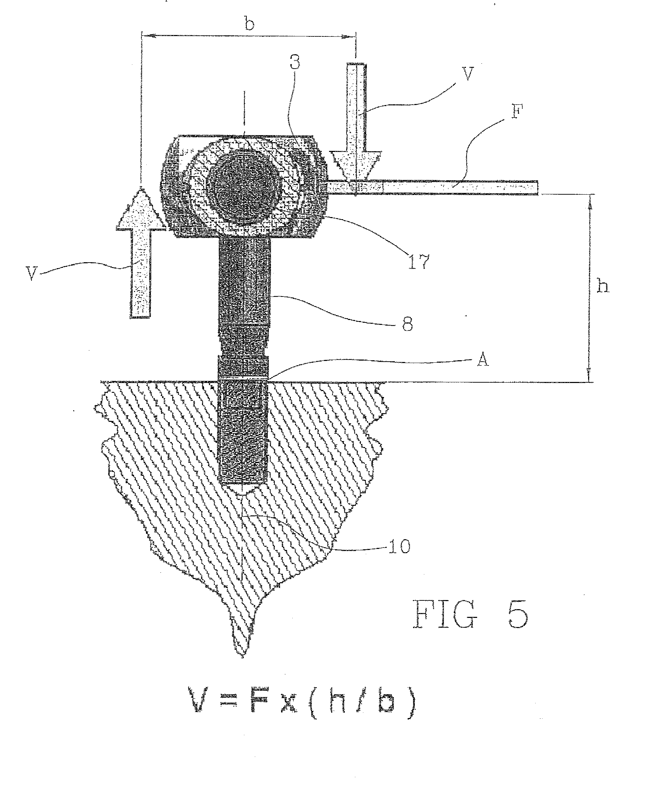

The device 1 is used in particular to enable optimal tightening of the screw 8 on the patient (see Figures 4 and 5).

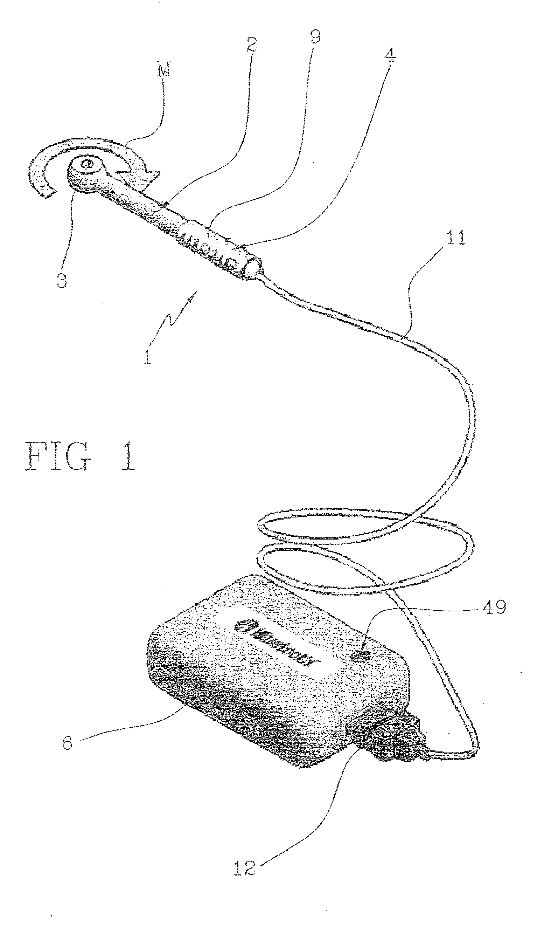

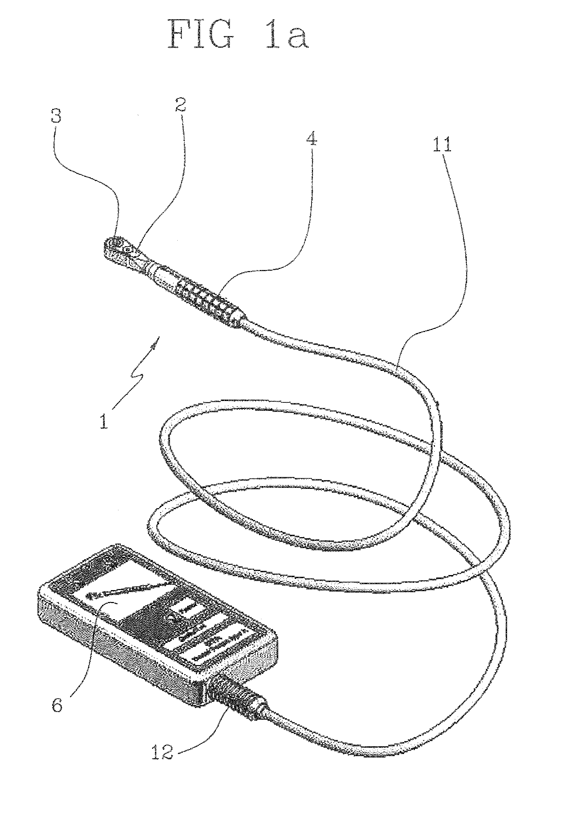

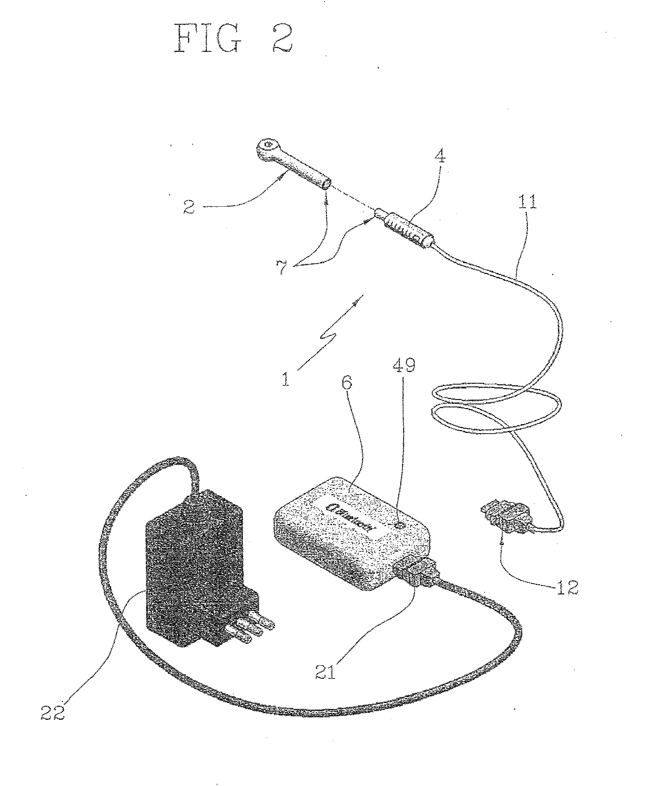

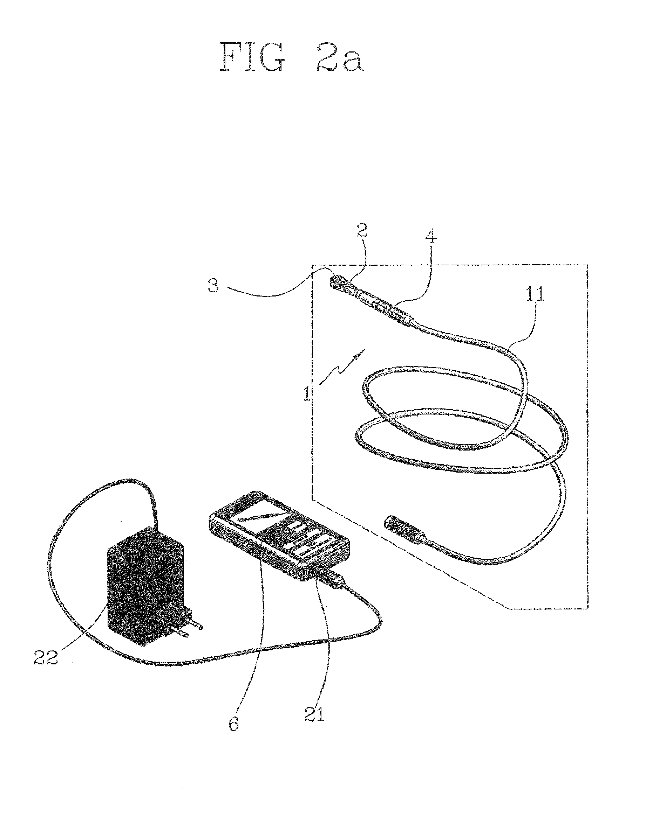

Going back to the device shown in Figures 1, 1a, 2 and 2a, said device comprises first of all at least a front portion 2 having a torque wrench 3 suitable for acting upon said screw for endosseous implants 8.

In particular, the front portion 2 comprises a ratchet wrench 3 with click, which can be freely rotated in only one direction.

In a first embodiment (Figures 1 and 2), the front portion 2 may consist in the dentist old mechanical wrench, modified if necessary in order to receive the rear portion 4 containing the electronic sensor system described below.

In this manner, the proposed device can be applied not only on new systems but also retrofit to existing tools, so that the user is not required to modify his or her standard operating procedure.

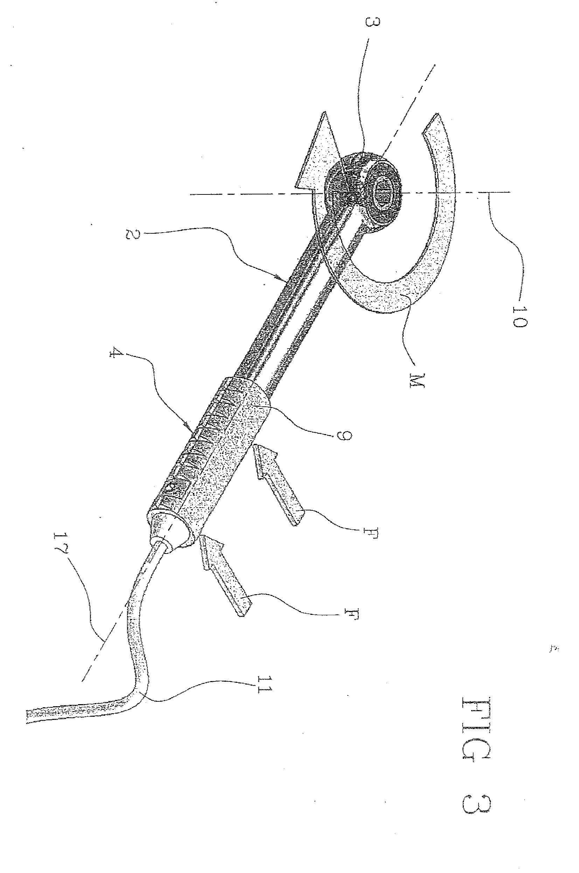

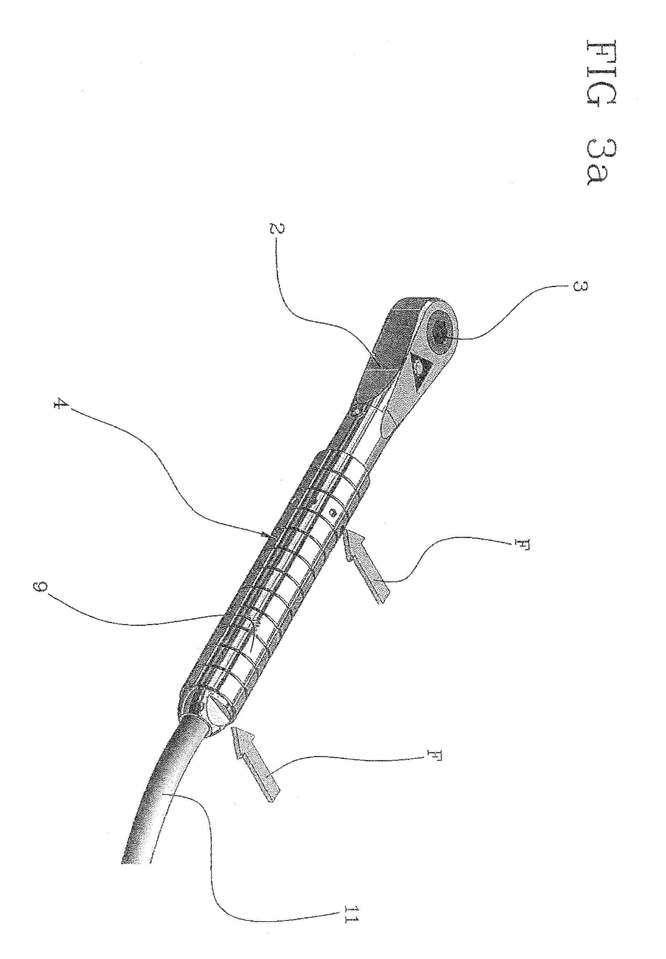

As is shown instead in Figures 3a and 4a, it is possible to construct the front portion 2 in such a manner that the coupling 63 can be quickly and simply changed so as to be adapted to any type of existing insert (hexagonal, square, etc...).

For such a purpose, the front and rear portions 2 can be removably engaged with each other, or else made in one piece and of a metallic material.

Again with reference to Figs. 3a and 4a, the front zone 2 will comprise a removable element 60 or cover that may be removably linked to the front portion 2 itself using suitable means 61, e.g. a tightening screw.

The front portion 2 will moreover be provided with a suitably shaped seat 62 designed to receive a replaceable coupling 63, so that the wrench 3 can be adapted to any type of existing insert (hexagonal, square, etc...).

Figure 4a illustrates two of such mutually replaceable inserts 63, one with a square head and the other one hexagonal.

Said insert 63 will generally have a lateral toothed surface to enable rotation in a single direction in combination with systems of a known type.

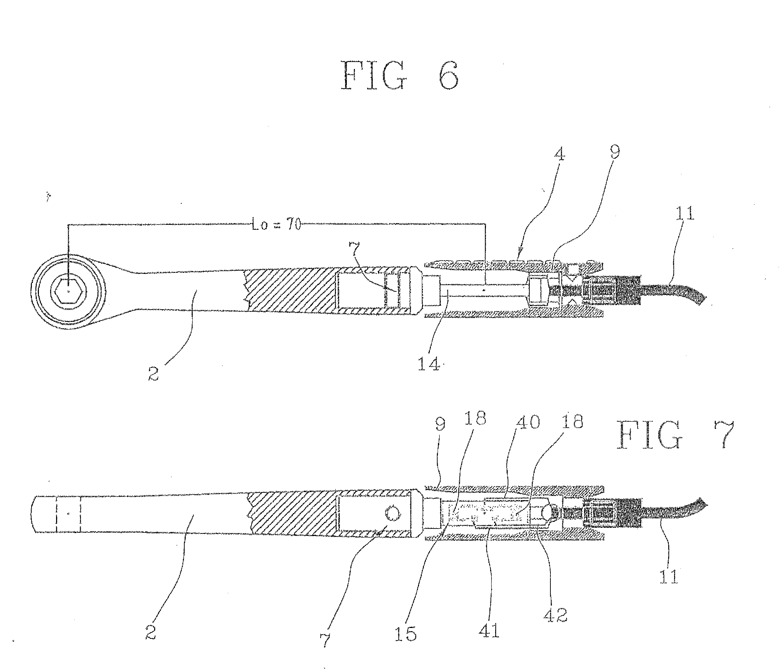

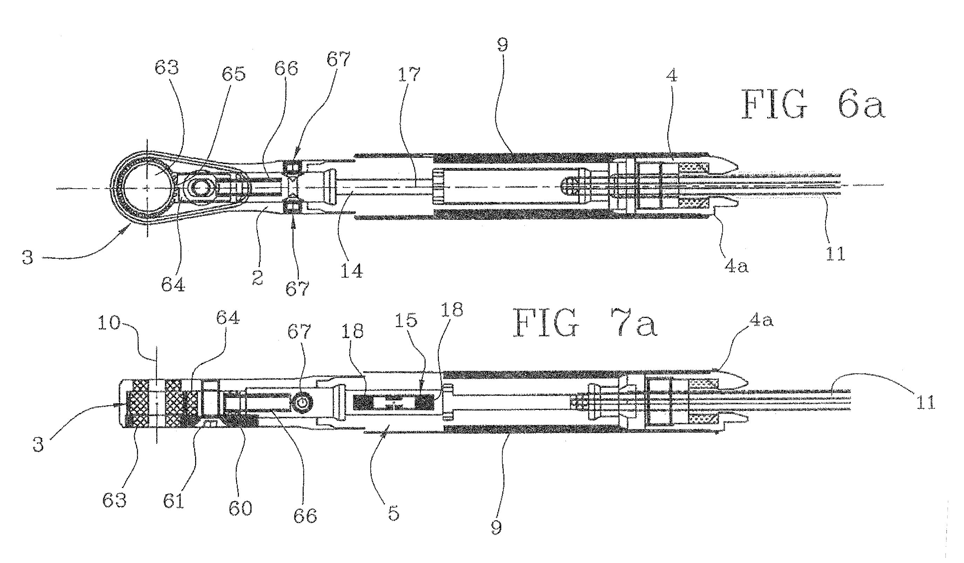

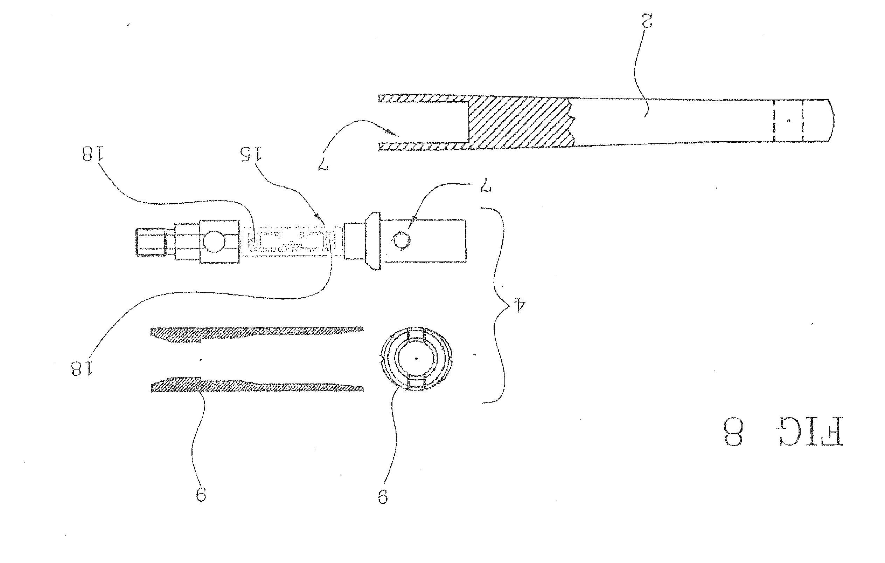

It should moreover be noted that whereas in the first embodiment the front portion 2 is separable from the rear portion 4 and can be sterilised in an autoclave, the second embodiment allows complete sterilisation of the wrench (in greater detail, autoclaveability of all parts potentially coming into contact with the patient in a surgical setting, and, therefore, the connection cable 11 and connector 12 as better explained below). In the first embodiment the rear portion 4, which is suitable for being manually actuated by the user, is, as noted, removably linked with the front portion, for example by means of a snap fit system or the like, as shown in Figures 7 and 8.

In any case, it will be possible to adopt any removable engagement means 7 which allows a stable link to be quickly achieved under the conditions of use of the device and easy separation of the front 2 and rear 4 portions in case of sterilisation or replacement of one of the portions.

Observing Figures 7 and 8, it can be noted that the rear portion 4 comprises a tubular body 9 with an elliptical section suitable for forming the handle of the device.

As is clearly visible in Figures 4 and 5, the elliptical section has its major diameter in a plane substantially orthogonal to the screwing axis 10 of the wrench 3.

The elliptical cross section is moreover worked over the two expansions of the major diagonal (see Figure 4) to allow a better grip and so as to counterbalance and cancel out (by virtue of both the geometry and the improved grip) the bending effect caused by misalignment between the points of application of the force F and the root of the implant.

Figures 4 and 5 illustrate how it is possible, by acting on the expansions of the tubular body 9, to cancel out the parasitic moment generated by the thrust force F, which is positioned on a different plane in relation to zone A, where the torque M is applied.

With reference to Figure 5, it may be noted that the user can apply two equal and opposite forces V at the lateral end portions of the tubular body 9 so as to cancel out (or substantially cancel out) the parasitic moment according to the mathematical-geometrical relationship shown in Figure 5.

Turning to Figures 6, 7 and 8, one may note the presence of at least a sensor 5 linked to the rear portion 4 and capable of detecting a deformation of the rear portion itself according to the torque M applied by means of the wrench 3.

In particular, the application of a force F on the handgrip generates said torque M around the rotation axis 10 and simultaneously deforms (in an imperceptible, but measurable manner) the device along its extension axis 17.

In detail, the rear portion comprises a shank 14 having a reduced section to which the sensor 5 is linked.

In reality the sensor 5 comprises at least one, and preferably two, strain gauges 15, 16 positioned on the shank 15 of reduced section on opposing surfaces substantially parallel to each other and lying in respective planes parallel to the screwing axis 10 of the wrench.

The strain gauges used are of the type with two grids 18 sensitive to variations in the length of the shank 14 along its extension axis.

Generally speaking, the strain gauges adopted are equivalent to each other.

Other types of sensors may obviously be used without departing from the scope of the invention.

Observing Figures 6a and 7a relating to the second embodiment, it should be noted that the tubular body 9 instead has a circular section suitably shaped on the exterior surface so as to increase the grip under conditions of use of the device itself.

The construction of the tubular body 9 from a metallic material is intended to enable the autoclaveability of said portion as well.

Again observing Figures 6a and 7a one notes the presence of the aforesaid coupling 63 or rotor suitably toothed on the outer surface and configured to cooperate with a device 64 which can allow movement of the rotor in a single direction of rotation by virtue of the presence of an appropriate tooth 65 suitable for cooperating with the outer teeth of the rotor for such purpose.

In the sectional view of Figure 7a one may also note the removable cover 60 and the fastening screw 61.

At the head of the ratchet wrench there is also present a pusher element or spring 66 capable of maintaining contact between said tooth 65 and the corresponding teeth of the rotor 63, thus assuring the transmission of force.

The embodiment illustrated in Figures 6a and 7a obviously also has a sensor 5 linked to the rear portion 4 and able to detect the deformation of the rear portion itself according to the torque M applied by means of the wrench 3.

In principle, this second embodiment behaves exactly like the previously described one and therefore detects the deformation of the device along its extension axis 17.

One may note in particular the shank 14 of reduced section to which the sensor 5 is linked.

It will obviously be possible to use the same sensors described previously with the same functions and positioning.

It should be noted, moreover, that the rear portion 4 is securely linked to the front portion 2 by adopting suitable linking means 67 defined by opposing grub screws evident in Figure 6a.

The rear portion 4 also has an output 4a, leading from which is a suitable connection cable 11 serving to supply power to said sensor 5 and to send a signal relative to the deformation detected to an external module 6.

It is clear that in order to enable the autoclaveability of the entire front portion 2 and rear portion 4 (including the connection cable 11 and connector 12) it is necessary for the inside of the tool to be completely sealed off from the outside environment using suitable gaskets and devices for such purpose.

Observing once more Figures 1, 1a, 2 and 2a it may be noted that the screwing device also comprises an external module 6 electrically connected to said sensor 5 in order to receive a signal S⁻ originating from said sensor at least during use of the device.

Using a suitable connection cable 11, generally an extremely resistant, highly flexible micro-cable so as not to obstruct the physician operations, it is possible to supply power to said sensor 5 (i.e. the strain gauges 15, 16) and to transfer the signal S⁻ originating from the sensor to the external module 6. To ensure the autoclaveability of the cable, the latter may be made of silicone; in any case it must be extremely flexible so as not to obstruct the physician operations.

The cable 11 has a connector 12 for a removable connection to the module 6.

In particular, Figure 7 shows the connection for the power supply 40 and the signal cables positioned at the ends of the strain gauge 41 and 42.

The same type of connection will obviously be adoptable in both embodiments (thus also with reference to Figures 6a and 7a).

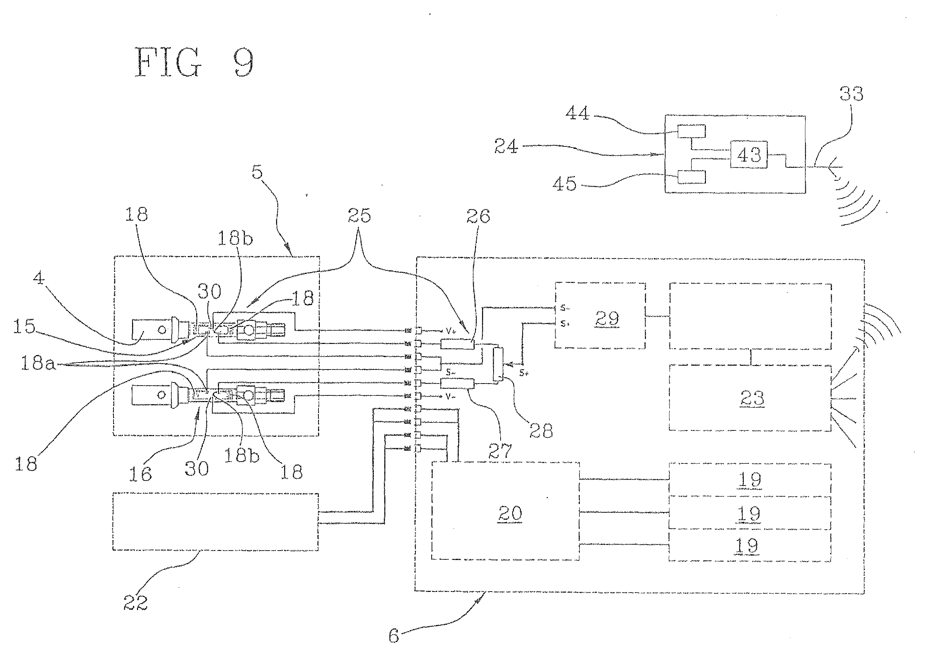

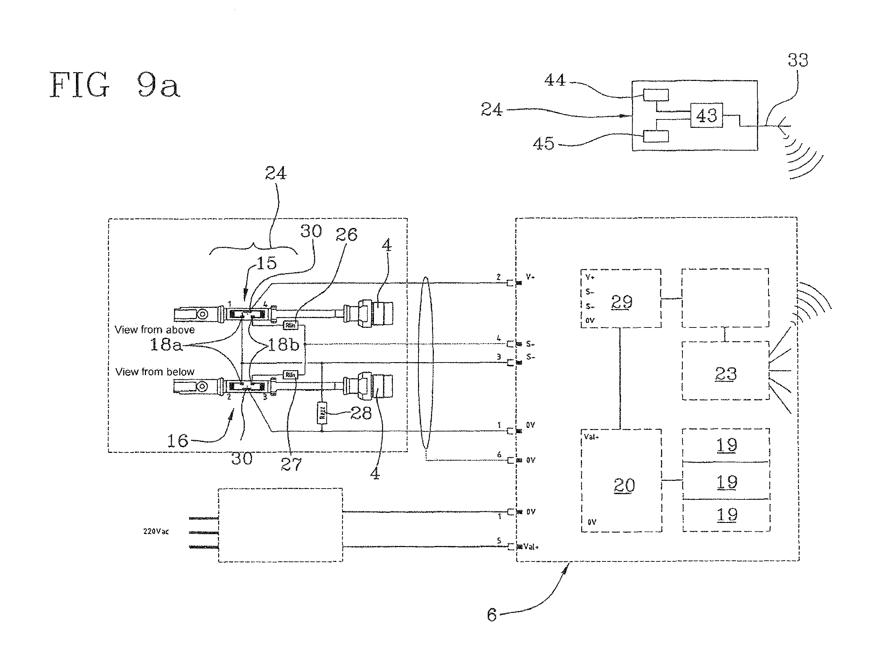

Going on to observe the external module 6, schematically represented in Figures 9 and 9a, it may be noted that said module comprises at least one NiMh battery 19 (and in general three batteries) in order to power the aforesaid sensor 5 and also comprises a recharging circuit 20 for recharging the battery.

It will generally be possible to use a normal battery charger 22, which will have a suitable connector 21 for interfacing with the external module 6 and powering the recharging circuit 20.

The connector 21 of the battery charger 22 will be engageable with the external module 6 only when the external module 6 is not connected to the rear portion 4.

This is mainly for safety reasons, in order to ensure that when the screwing device is being used for implanting the screw 8 there will be no high-voltage connection to the medical device.

To this end, the two connectors 12, 21 can be externally identical and, obviously, once inserted (one or the other, alternatively) into the corresponding seat of the module 6, each of the two will interface with the respective contacts for the exchange of data (sensor and sensor power supply) or for recharging the battery (battery charger) as electrically represented in Figure 9.

The external module 6 further comprises a wireless transmission module 23, preferably of the Bluetooth type, though obviously any type of wireless communication can be used, in order to send the signal originating from the sensor 5, generally already processed, to an external processor 24 equipped with a corresponding wireless module 33.

In general, as better explained below, the signal S⁻ originating from the strain gauges 15, 16 is processed within the external module 6 and the torque computed (or the electric signal proportional thereto) is wirelessly transmitted to an external electronic processor 24, which usually comprises a control unit 43, for example a microprocessor, and a suitable memory 44 associated with the control unit in a known manner. A suitable program executed by the control unit 43 can memorise and use the signal proportional to the torque applied and detected in order to provide suitable indications, information and certifications to the user.

In particular, the program comprises at least a memory module for storing the torque data received, at least a display module and at least a printer module for printing out the torque value (or a signal corresponding thereto) as a function of time.

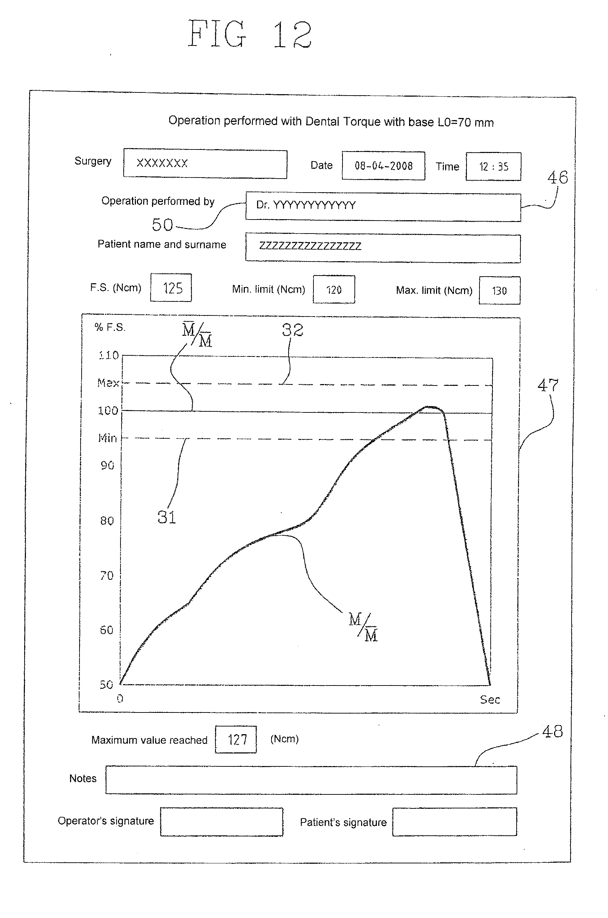

As shown in Figure 12, when the program is opened a screen appears in which there is a box for entering the name of the physician 50 and a box for entering the name of the patient 46, as well as the date and time. The torque to be guaranteed $\overline{M}$ (also called F.S. full scale in Ncm) is then set.

The minimum and maximum acceptance limits, indicated with the numerical references 32, 31 in the appended Figure, are likewise set.

The graph 47 represents the execution times (with the possibility of auto-ranging) on the X axis and the measured torque values M on the Y axis; the torque is monitored starting from 50% of the full scale value $\overline{M}$.

Finally, a blank space 48 is present for the entry of comments or notes.

The working sequence consists in preparing the ratchet wrench with the front 2 and rear 4 portions mutually linked and the accessories suitable for the operations to be performed.

Via the connector 12 the front and rear portions are connected to the external module 6.

The device is switched on by means of an on-off pushbutton 49 that must light up.

At this point the screwing operations can begin and, as the torque M applied on the screw 8 progressively increases (in general when the value reaches about 70% of the full scale value), audible signalling means 45 associated with the external module 6 will generate a beep.

This sound will increase in frequency until becoming continuous when the torque M reaches the full scale $\overline{M}$, i.e. the set, desired torque.

All values of the torque applied are recorded at a frequency of 10 points per second so that they can be displayed in the diagram 47.

For the sake of simplicity and interest, the memorisation of the measured data automatically starts and ends at 50% of the full scale; this makes it possible to obtain a single diagram, without taking account of all the initial low-load adjustment operations that are of no interest for the purpose of validating the operations.

As noted, the memorization time is tied to the exceeding of the 50% threshold and the display is auto-ranging.

Obviously, all of the above-mentioned values are initially set as default values but may edited via software.

Once the operation has been completed, it will be possible to print out the document certifying the operation performed, for example a document of the type illustrated in Figure 12.

With respect to the external module 6, when the latter signals that the battery charge level is low or when it is decided to recharge the batteries, it will be necessary first of all to disconnect the connector 12 (and this will rule out any possibility of the wrench being connected to the mains power supply).

To recharge the internal batteries it will be necessary to connect the connector 21 of the battery charger 22 and leave them to charge for around 2 hours.

It should be noted that the two embodiments described above (Figs. 7 and 7a) can be alternatively coupled with external modules differing from the type illustrated in Figures 9 and 9a.

With reference to the wiring diagram shown in Figure 9, it may be noted that the coupling between the rear portion 4 and the external module 6 generates, from an electrical viewpoint, a bridge circuit 25 having at least a first and a second resistor 26, 27 and an element for regulating the electrical values of the circuit or trimmer 28, circuitally interposed between said resistors 26, 27.

The bridge consists in particular of the two aforesaid strain gauges 15 and 16, the two resistors 26 and 27 and the aforesaid trimmer 28.

In particular, the power supply is delivered to an intermediate point 30 between the two grids 18 of each of the strain gauges; one corresponding end 18a of each strain gauge 15, 16 is circuitally connected to the input side of a signal amplifier 29; the other end 18b of each strain gauge 15, 16 is connected to the input side respectively of one 26 and the other 27 resistor situated on the external module.

The trimmer 29 can be adjusted a priori so as to produce a condition of equilibrium in the bridge and render the measurement of the torque M independent from the point of application of the force F along the extension axis 17 of the device.

In particular, the overall value in Ohms of the resistors 26, 27 and the trimmer 28 will be a function of the distance $L_0$ between the strain gauges and the point of application A of the torque M.

With reference to Figure 9a it may be noted that the bridge circuit 25 is instead totally defined within the wrench, and in particular within the rear zone 4, so that the electronic module 6 directly receives the signal S⁻ to be amplified and then sent to the electronic processor 24.

In this second embodiment, the electric power connections to the strain gauges 15 and 16 are identical, as are the connections of the latter to the resistors 26 and 27.

In this case, however, the resistor, which may be adjusted during calibration of the tool, Razz 28, is interposed between the branch of the bridge carrying the signal S⁻ and the 0V ground connection. The resistor 28 has the same purpose as the previously described trimmer 28.

Based on a plurality of experiments it has been possible to calculate the relationship that links the overall trim value to the distance $L_0$ between the point of application of the strain gauges and the axis A of application of the torque, i.e.:

$$T_C = 10162 \times L_0^{-1.0567}$$

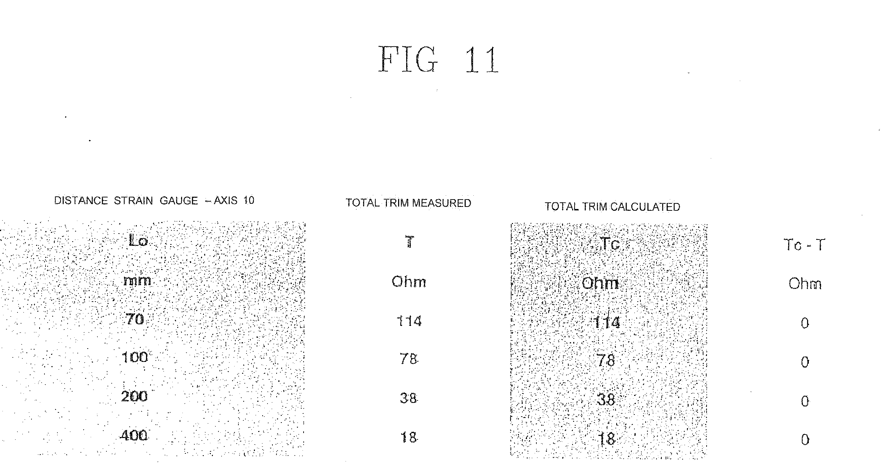

The data of the table provided in Figure 11 show the total trim values measured (second column) and the total trim values calculated according to the above formula (third column) for different positions of the strain gauges identified by the different distances $L_0$.

As may be noted, the values match perfectly.

Therefore, once the strain gauges have been duly positioned and the distance from the centre of application of the torque is known, it will be possible to set the device accordingly so that it can generate torque values independent from the point of application of the force along the device axis, thus assuring a precise, reliable and independent measurement.

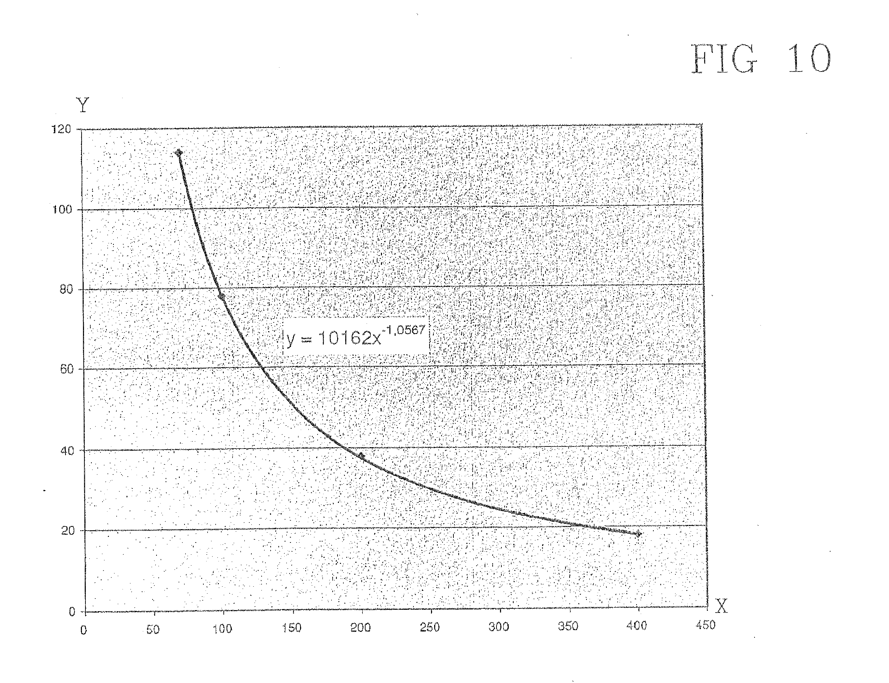

It should be noted that the relationship is also stated and represented in the appended Figure 10.

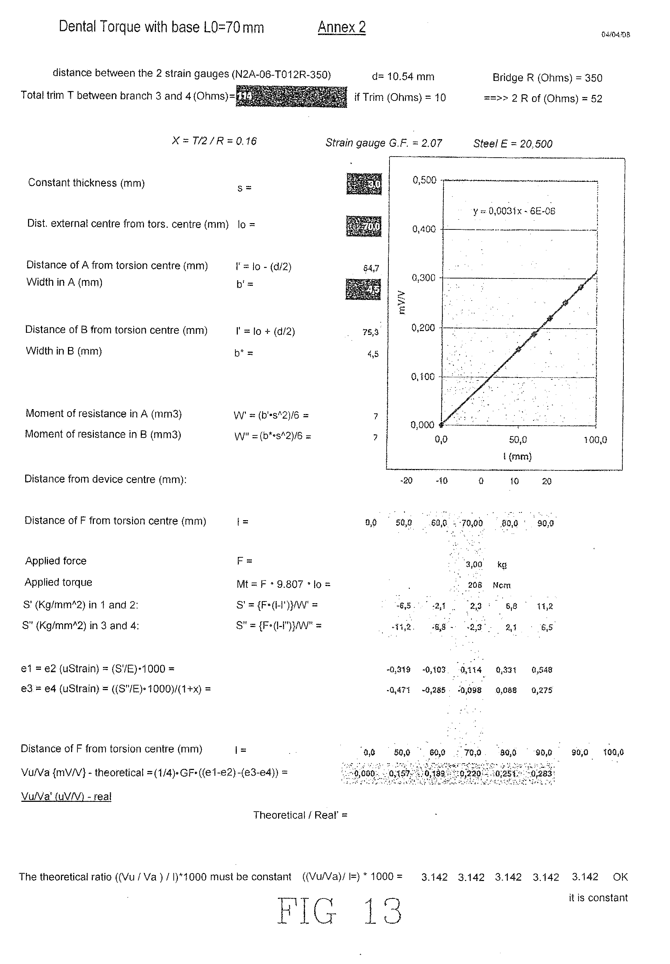

Finally, the table appended hereunder (Figure 13) shows the deformation calculations recorded by the device according to the invention when a force equal to 3 kilograms was applied in different positions along the device axis, i.e. 50, 60, 70, 80 and 90 millimetres from the centre of torsion.

The experiment was performed using two strain gauges manufactured by Vishay Micro-Measurements and identified by the codes N2A-06-T012R-350.

As may be noted, the theoretical ratio calculated (Vu/va)/L proves to be constant (i.e. independent from the point of application of the force).

It should moreover be noted that the formulas adopted also apply for distances $L_0$ which vary considerably, thus attesting to the considerable precision of the device.

The invention achieves some major advantages.

First of all, the screwing device according to the invention makes it possible to reutilise the old mechanical wrench by making simple changes in order to associate the electronic device with it; as a result, the proposed device can be applied not only on new systems but also retrofit to existing tools, so that the user is not required to modify his or her standard operating procedure.

The tool realised is precise and stable over time while maintaining the ease of handling and dimensional characteristics of a mechanical torque ratchet wrench. In addition, the possibility of recording data during the operation enables certification thereof, thus assuring that the operation is carried out correctly. In other words, the tool is capable not only of performing the operations for which it was designed but also of providing substantial evidence (e.g. a certifying document) of the operations carried out. Furthermore, all parts of the device that may possibly come into contact with patients are autoclaveable and it may therefore be used in surgical settings.

In addition, the efficiency of the system can be easily and continuously monitored: in fact, when the ratchet wrench is maintained in a horizontal position and locked on the ratchet side, the weight of the electronic module left hanging freely in a vertical position gives a reading (in Ncm for each system) whose constancy over time within a predefined range guarantees the perfect functionality of the system.

It is in fact sufficient to lift the module in order to have a weight of 0 on the ratchet and thus clear the reading, then allow the module to dangle and take a reading in static conditions and check whether the value falls within the established range. Notwithstanding all of the characteristics and advantages described above, the torque wrench is easily sterilisable, lightweight and also easy to handle.

**Claims**

1. A screwing device for medical applications, in particular for endosseous implantology also in a surgical setting, comprising:

   - at least a front portion (2) having a torque wrench (3) suitable for acting upon a suitable screw (8);
   - at least a rear portion (4) suitable for being manually actuated by a user, said front (2) and rear (4) portions being reciprocally linked under conditions of use of the device; and
   - at least one sensor (5) linked to said rear portion (4) in order to detect a deformation of the rear portion, which will be a function of the torque (M) applied to the screw by means of said wrench (3), **characterised in that** it further comprises an external module (6) connected to said sensor (5) in order to receive a signal (S⁻) originating from said sensor at least during use of said device and **in that** said front and rear portions (2), (4) are configured and coupled in such a way as to be autoclaveable.

2. The device according to the preceding claim, **characterised in that** the front portion (2) comprises a ratchet wrench (3) with clicks, which can be freely rotated in one direction only.

3. The device according to any of the preceding claims, **characterised in that** it comprises a connection cable (11) for connecting the rear portion (4) to the external module (6), said cable (11) having a connector (12) for a removable connection to said module (6) and said cable and said connector being structured and coupled to each other and to the rear portion (4) in such a way as to be autoclaveable.

4. The device according to the preceding claim, **characterised in that** the connection cable (11) carries an electric power supply from the external module (6) to said sensor (5) and the signal (S⁻) originating from the sensor to the external module (6).

5. The device according to any of the preceding claims, **characterised in that** the rear portion (4) comprises a shank (14) having a reduced section, the sensor (5) being positioned on said shank (14), the sensor (5) comprising at least one, and preferably two, strain gauges (15), (16), said strain gauges (15), (16) being positioned on said shank (14) of reduced section on opposing surfaces substantially parallel to each other and lying in respective planes substantially parallel to the screwing axis (10) of the wrench.

6. The device according to any of the preceding claims, **characterised in that** the external module (6) comprises at least a battery (19) for powering said sensor (5) and preferably a battery recharging circuit (20), and a battery charger (22) having a connector (21) for interfacing with the external module and powering the recharging circuit, in particular the connector (21) of the battery charger (22) being engageable with the external module (6) only when the external module (6) is not connected to the rear portion (4).

7. The device according to any of the preceding claims, **characterised in that** the external module (6) comprises a wireless transmission module (23), preferably of the Bluetooth type, for sending the signal originating from the sensor (5), and processed if necessary, to a processor (24).

8. The device according to any of the preceding claims, **characterised in that** each of said strain gauges (15), (16) is powered at an intermediate point (30) between the two grids (18), one corresponding end (18a) of each strain gauge (15), (16) being circuitally connected to the input side of the signal amplifier (29) and the other end (18b) of each strain gauge (15), (16) being connected to an input respectively of one (26) and the other (27) resistor placed on the external module (6).

9. The device according to any of the preceding claims, **characterised in that** it comprises a control unit (43) and a memory (44) associated with the control unit, a program being executed by the control unit (43), said program comprising:

   - at least a memory module for storing the torque (M) data received; and
   - at least a module for displaying and/or printing out the torque value or a signal corresponding thereto as a function of time;
   preferably, the program further comprises a setting module for setting the desired torque value ($\overline{M}$) to be reached and for setting, if necessary, acceptable upper (32) and lower (31) limits.

10. The device according to any of the preceding claims, **characterised in that** the front portion (2) further comprises a removably linked coupling (63), said coupling (63) having a suitable engaging seat for rotatably operating an insert, said coupling (63) being replaceable with other couplings having different engaging seats.

FIG 1

# FIG 1a

FIG 2

# FIG 2a

FIG 3

FIG 3a

FIG 4

EP 2 221 022 A1

FIG 4a

FIG 5

$$V = F \times (h/b)$$

EP 2 221 022 A1

# FIG 6

# FIG 7

FIG 7a

FIG 6a

FIG 8

FIG 9

EP 2 221 022 A1

FIG 9a

$$y = 1016.2x^{-1.0567}$$

FIG 10

FIG 11

| DISTANCE STRAIN GAUGE – AXIS 10 | | TOTAL TRIM MEASURED | TOTAL TRIM CALCULATED | |
| --- | --- | --- | --- | --- |
| Lo | mm | T · Ohm | Tc · Ohm | Tc - T · Ohm |
| 70 | | 114 | 114 | 0 |
| 100 | | 78 | 78 | 0 |
| 200 | | 38 | 38 | 0 |
| 400 | | 18 | 18 | 0 |

# FIG 12

Operation performed with Dental Torque with base L0=70 mm

Surgery | XXXXXXX | Date | 08-04-2008 | Time | 12 : 35

46

Operation performed by | Dr. YYYYYYYYYYYY

50

Patient name and surname | ZZZZZZZZZZZZZZZZZZ

F.S. (Ncm) | 125 | Min. limit (Ncm) | 120 | Max. limit (Ncm) | 130

% F.S.

$\overline{M}/\overline{M}$  32

110

Max

100

Min

90

80

31

70

$M/\overline{M}$

60

50

0 — Sec

47

Maximum value reached | 127 | (Ncm)

48

Notes

Operator's signature | Patient's signature

25

Dental Torque with base L0=70 mm          <u>Annex 2</u>

04/04/08

distance between the 2 strain gauges (N2A-06-T012R-350)      d= 10.54 mm        Bridge R (Ohms) = 350

Total trim T between branch 3 and 4 (Ohms)= ▓▓▓      if Trim (Ohms) = 10      ==>> 2 R of (Ohms) = 52

$X = T/2 / R = 0.16$          Strain gauge G.F. = 2.07        Steel E = 20,500

| | | | |
|---|---|---|---|
| Constant thickness (mm) | s = | ▓30 | |
| Dist. external centre from tors. centre (mm) | lo = | ▓700 | |
| Distance of A from torsion centre (mm) | l' = lo - (d/2) | 64,7 | |
| Width in A (mm) | b' = | ▓45 | |
| Distance of B from torsion centre (mm) | l' = lo + (d/2) | 75,3 | |
| Width in B (mm) | b* = | 4,5 | |
| Moment of resistance in A (mm3) | $W' = (b' \cdot s^2)/6 =$ | 7 | |
| Moment of resistance in B (mm3) | $W'' = (b* \cdot s^2)/6 =$ | 7 | |

y = 0,0031x - 6E-06

mV/V vs l (mm)

Distance from device centre (mm):

| Distance of F from torsion centre (mm) | l = | 0,0 | 50,0 | 60,0 | 70,00 | 80,0 | 90,0 |
|---|---|---|---|---|---|---|---|
| Applied force | F = | | | | 3,00 | kg | |
| Applied torque | $Mt = F \cdot 9.807 \cdot lo =$ | | | | 206 | Ncm | |
| S' (Kg/mm^2) in 1 and 2: | $S' = \{F \cdot (l-l')\}/W' =$ | -6,5 | -2,1 | 2,3 | 6,8 | 11,2 | |
| S" (Kg/mm^2) in 3 and 4: | $S'' = \{F \cdot (l-l'')\}/W'' =$ | -11,2 | -6,8 | -2,3 | 2,1 | 6,5 | |
| e1 = e2 (uStrain) = (S'/E)·1000 = | | -0,319 | -0,103 | 0,114 | 0,331 | 0,548 | |
| e3 = e4 (uStrain) = ((S"/E)·1000)/(1+x) = | | -0,471 | -0,285 | -0,098 | 0,088 | 0,275 | |

| Distance of F from torsion centre (mm) | l = | 0,0 | 50,0 | 60,0 | 70,0 | 80,0 | 90,0 | 90,0 | 100,0 |
|---|---|---|---|---|---|---|---|---|---|
| Vu/Va {mV/V} - theoretical =(1/4)·GF·((e1-e2)-(e3-e4)) = | | 0,000 | 0,157 | 0,189 | 0,220 | 0,251 | 0,283 | | |

<u>Vu/Va' (uV/V) - real</u>

Theoretical / Real' =

The theoretical ratio ((Vu / Va ) / l)*1000 must be constant    ((Vu/Va)/ l=) * 1000 =    3.142   3.142   3.142   3.142   3.142   OK

it is constant

FIG 13

Europäisches
Patentamt

European
Patent Office

Office européen
des brevets

**EUROPEAN SEARCH REPORT**

Application Number

EP 09 16 7406

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 20 2007 012866 U1 (RECK ENGINEERING INGENIEURBUER [DE]) 17 January 2008 (2008-01-17) * paragraphs [0011], [0012]; claims 1-15 * | 1-7,9,10 | INV. A61C8/00 B25B23/142 |
| A | EP 0 362 696 A (SNAP ON TOOLS CORP [US]) 11 April 1990 (1990-04-11) * column 4, lines 40-58 * * column 5, lines 22-37; figures 1-4,7 * | 1,5,8 | |
| A | DE 10 2005 039682 A1 (STAND TOOLS ENTPR CO LTD TAICH [TW]) 30 March 2006 (2006-03-30) * paragraphs [0021], [0024]; figures 1,2 * | 1,5 | |
| A | US 4 958 541 A (ANNIS JEFFREY R [US] ET AL) 25 September 1990 (1990-09-25) * column 3, lines 37-45; figures 1-3 * | 1,5,8 | |
| A | US 4 006 629 A (BARRETT GARY L ET AL) 8 February 1977 (1977-02-08) * figures 1,3 * | 1,8 | TECHNICAL FIELDS SEARCHED (IPC) A61C B25B |

The present search report has been drawn up for all claims

| Place of search | Date of completion of the search | Examiner |
|---|---|---|
| Munich | 18 November 2009 | Roche, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 16 7406

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2009

| Patent document cited in search report | | Publication date | Patent family member(s) | | Publication date |
|---|---|---|---|---|---|
| DE 202007012866 U1 | | 17-01-2008 | NONE | | |
| EP 0362696 | A | 11-04-1990 | CA | 1324506 C | 23-11-1993 |
| | | | DE | 68911101 D1 | 13-01-1994 |
| | | | DE | 68911101 T2 | 17-03-1994 |
| | | | US | 4982612 A | 08-01-1991 |
| DE 102005039682 A1 | | 30-03-2006 | TW | 276516 B | 21-03-2007 |
| US 4958541 | A | 25-09-1990 | CA | 2023106 A1 | 14-04-1991 |
| US 4006629 | A | 08-02-1977 | NONE | | |

EPO FORM P0459

For more details about this annex : see Official Journal of the European Patent Office, No. 12/82

**EP 2 221 022 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4397196 A
- US 7313990 B
- US 200627058 B
- US 4864841 A